# EUROPEAN PATENT SPECIFICATION

(11) **EP 2 303 223 B1**
(45) Date of publication and mention of the grant of the patent: **04.07.2018**
(21) Application number: 09800030.0
(22) Date of filing: 10.07.2009
(51) Int. Cl.: A61K 8/70, A61K 8/06, B01F 17/14

(54) **MODIFIED OILS AND MULTIPLE EMULSIONS**
MODIFIZIERTE ÖLE UND MEHRFACHEMULSIONEN
HUILES MODIFIÉES ET ÉMULSIONS MULTIPLES

(30) Priority: 23.07.2008 IT MI20081339
(43) Date of publication of application: 06.04.2011
(73) Proprietor: Giuliani S.p.A., 20129 Milano (IT)
(72) Inventor: GIULIANI, Giammaria, I-20100 Milan (IT); PANTINI, Giovanni, I-20134 Milan (IT); MASCOLO, Antonio, I-20126 Milan (IT); BENEDUSI, Anna, I-20124 Milan (IT)
(74) Representative: Coppo, Alessandro
(86) International application number: PCT/EP2009/058821
(87) International publication number: WO 2010/009989

(56) References cited:
- EP-A- 1 074 243
- EP-A- 1 145 722
- WO-A-2005/049089
- PANTINI ET AL: "Perfluoropolyether phosphate : a non-irritating acidic agent of cosmeceutical use" CLINICS IN DERMATOLOGY, J.B. LIPPINCOTT, PHILADELPHIA, PA, US, vol. 26, no. 4, 1 July 2008 (2008-07-01), pages 387-391, XP023610836 ISSN: 0738-081X [retrieved on 2008-08-05]
- KIZLING J ET AL: "On the formation and stability of high internal phase O/W emulsions" ADVANCES IN COLLOID AND INTERFACE SCIENCE, ELSEVIER, vol. 123-126, 16 November 2006 (2006-11-16), pages 295-302, XP025061753 ISSN: 0001-8686 [retrieved on 2006-11-16]

## Description

### Technical Field

The present invention concerns modified oils and multiple emulsions. The present invention relates to oil-in-water (O/W) emulsions with high internal phase and is mainly used in the cosmetic field and for pharmaceutical formulations.

### Background of the Invention

It is convenient to distinguish between conventional emulsions and various types of non conventional emulsions, in particular multiple emulsions, hyperfluid and superfluid emulsions, and high internal phase emulsions (HIPE).

### Conventional emulsions

As it is well-known, emulsions are dispersions of one liquid (the dispersed phase or internal phase) in another liquid, wherein the first liquid is non miscible (continuous phase).

The dispersions are commonly obtained by supplying energy to the system while stirring and generally operating with the two phases being heated.

Devices can be used for this purpose, which can be very different from one another; among these, there are rotor/stator mixers; turboemulsifiers variously shaped and equipped (with mixers, stirrers, counter-stirrers, scrapers, and fusers aimed at heating the lipophylic phase); high dynamic pressure homogenizers, and so on.

In the production and stabilization of the emulsions, emulsifying agents are commonly used, generally surfactant agents, whose function is to lower the interface tension between the two liquid phases.

Generally, one phase is constituted by water, in which water-soluble ingredients are dissolved, while the other phase is of oily nature. It is possible to obtain dispersions of oils in water, named oil-in-water (O/W) emulsions, or, but less often, dispersions of an aqueous solution in an oily phase, named water-in-oil (W/O) emulsions. In both cases, the internal phase is generally less than the continuous phase; however, and still remaining in the conventional emulsions field, it is possible to have emulsions with more internal phase than external phase, getting close to the critical limit of 74% by volume, beyond which there are obvious geometrical constraints.

Independently from the relation between the two phases, conventional emulsions are unstable thermodynamically due to the tendency of the internal phase to coalescence up to the separation of the two phases by stratification. This phenomenon does not prevent the commercial use of emulsions, since coalescence occur usually after a time much longer than the products market life.

Besides coalescence, there is another mechanism that determines an alteration of the emulsions. This second mechanism depends on the relation between the density of the internal phase and the external phase.

Different densities cause accumulation of the internal phase i) in the upper part, if the internal phase is lighter than the external one (creaming) or ii) in the lower part, if the internal phase is heavier (sedimentation).

In both cases, the phenomenon is reversible: generally, a moderate shaking, even by the user before the use, is enough to reconstitute the initial state. Furthermore, according to the Stokes' law this form of instability can be avoided by increasing the emulsion viscosity and reducing the dimensions of the dispersed particles.

### Non conventional emulsions

### Multiple emulsions

Multiple emulsions (called also double emulsions) are constituted by dispersions of simple emulsions, of the O/W or W/O type, in a continuous liquid phase, that can be an oil or water. In the first case, O/W/O emulsions are produced.

In the second case, more common, the emulsions are dispersed in water with the formation of W/O/W emulsions.

Actually, W/O/W emulsions combine the better sensory properties (minor oiliness) of O/W emulsions with the better moisturizing capacity and persistence of W/O emulsions. Besides, the two separate aqueous phases allow the formulation of active principles, which are poorly compatible or have stability at different pH. However, multiple emulsions have not become of common use because of their preparation constraints. They are usually prepared in two steps. In the first one, a very stable primary emulsion is obtained (generally of the W/O type), through the addition of electrolytes (sodium or magnesium salts) in the aqueous phase and the use of specific emulsifying agents in relation to oils.

In the second step, the primary emulsion is dispersed in water, with a weaker mechanical action and, preferably, with the help of hydrophilic polymeric emulsifiers, in a 40-60% ratio with reference to the overall emulsion.

This ratio, together with the choice of the emulsifying agents, depending on polarity of the oily phase, the ionic force of the internal aqueous phase, the viscosity of the two aqueous phases, are among the critical parameters for improving the system, which however remains poorly stable, which is the main obstacle to the commercial success of these emulsions.

### Hyperfluid and superfluid emulsions

In the literature, emulsions with viscosity comprised between 500 and 2000 mPa/s are normally classified as "fluid", while emulsions with viscosity near to 100 mPa/s are defined "hyperfluid".

Less common, the term "superfluid emulsions" describes highly fluid emulsions, with viscosity lower than 10 mPa/s, generally in the range of 2÷4 mPa/s. Due to the fluidity, these emulsions tend strongly to creaming (when oils have lower density than water) or to sedimentation (with heavier oils).

### HIPE emulsions

Emulsions with the internal phase over 74% by volume are named "High Internal Phase Emulsions" (HIPE). As conventional emulsions, also HIPE emulsions are of the O/W or W/O type and unstable from the thermodynamic point of view. These emulsions present deformed micellar structures, since it is impossible to maintain the spherical shape with so reduced volumes of the continuous phase.

In the most common HIPE emulsions of the O/W type, the external (aqueous) phase has a density generally near to 1.0 g/ml, while the internal (oily) phase has a density that can vary between 0.85 (hydrocarbons) and 1.05 (solar filters) g/ml. It follows that a HIPE emulsion of the O/W type has a percentage content by volume of the oily phase generally a few units greater than the content by weight, while only in few cases the contrary occurs, besides, with a lower percentage incidence.

With their high content of the internal phase, which can reach even 90% and more, these emulsions are very viscous and difficult to prepare with a limited choice of oils, mainly of apolar type (silicone oils and hydrocarbons are the most common).

### Summary of the invention

At present, the need exists to have emulsions and modified oils, suitable for applications in cosmetic and pharmaceutical field, which can be used as finished products or as intermediates of formulation by dilution in water, to obtain fluid emulsions, or by dispersion in the aqueous phase of more complex compositions, overcoming the problems of the active substances, such as stability, compatibility, and bioavailability.

One of the main objects of the present invention is then to provide oil-in-water (O/A) emulsions with high internal phase to be used as modified oils, which present lipophobicity (oil repellence) and homophobicity (self-repellence tendency), combined with balanced (limited) hydrophilicity/hydrophobicity (water affinity/repellence).

Another object of the present invention is to provide a method for preparing oil-in-water HIPE emulsions and modified oils that find use applications in cosmetic field and whose realization does not require high production costs.

In view of these objects, there is provided, according to a first aspect of the present invention, an oil-in-water (O/W) HIPE emulsion comprising an aqueous continuous phase and an oily internal phase of more than 74% by volume, dispersed in the continuous phase, said emulsion being characterized in that it is lipophobic and homophobic and it includes perfluoropolyether phosphate (PFPE phosphate), with INCI name "Polyperfluoroethoxymethoxy difluoroethyl PEG phosphate", of the formula:

(HO)₂OP-O-(CH₂CH₂O)ₙ-CH₂-R_{f}-CH₂-(OCH₂CH₂)ₙ-O-P-O(OH)₂

wherein:
n from 1 to 2 (1÷2)
R_{f} is a perfluoropolyether chain of formula:

   -(CF₂-CF₂O)ₚ-(CF₂O)_{q}-

   with p/q from 0.5 to 3.0 (0.5÷3.0)
   the average molecular weight of R_{f} is comprised from 500 to 4000,
   preferably from 1000 to 2000, more preferably from 1400 to 1600.
solubilised in the continuous aqueous phase. Other characteristics of the oil-in-water (O/W) HIPE emulsion or of modified oils, having lipophobicity and homophobicity of the invention, are reported in the enclosed claims 3-8.

It has been surprisingly noticed that, an oil or a mixture of oils acquires, through the treatment with PFPE phosphate solubilized in aqueous solution, with the transformation into a HIPE emulsion, typical characteristics of the high fluorine compounds, in particular lipophobicity and homophobicity, in addition to anti-sticking properties, that turn into non miscibility of oils (or mixtures of oils) treated separately with PFPE phosphate and improved sensory qualities.

According to another aspect of the invention, there are provided uses of an oil-in-water HIPE emulsion, as indicated in claims 9, 10.

According to another aspect of the invention, there is provided a method for preparing an oil-in-water O/W emulsion, with high internal phase (HIPE), having an oily internal phase of more than 74% by volume, comprising the dispersion of an oil in an aqueous solution of a PFPE phosphate of formula:

(HO)OP-O-(CH₂CH₂O)ₙ-CH₂-Rf-CH₂-(OCH₂CH₂)ₙ-O-P-O(OH)₂

wherein:
n from 1 to 2
R_{f} is a perfluoropolyether chain of formula:

   -(CF₂-CF₂O)ₚ-(CF₂O)_{q}-

   with p/q from 0.5 to 3.0
   the average molecular weight of R_{f} is comprised from 500 to 4000, preferably
   from 1000 to 2000, more preferably from 1400 to 1600.

According to another aspect of the invention, there is provided the use of a PFPE phosphate of formula:

(HO)₂ OP-O-(CH₂CH₂O)ₙ-CH₂-R_{f}-CH₂-(OCH₂CH₂)ₙ-O-P-O(OH)₂

wherein:
n from 1 to 2
R_{f} is a perfluoropolyether chain of formula:

   -(CF₂-CF₂O)ₚ-(CF₂O)_{q}-

   with p/q from 0.5 to 3.0
   the average molecular weight of R_{f} is comprised from 500 to 4000,
   preferably from 1000 to 2000, more preferably from 1400 to 1600.
solubilised in aqueous solutions, containing, if necessary, a polar solvent, for preparing an oii-in-water HIPE emulsion, in which the internal phase amounts to at least 74% by volume and presents typical characteristics of the high fluorine content compounds, in particular lipophobicity and homophobicity. Cosmetic compositions comprising perfluoropolyether phosphates are known from EP 1145722 A1 and EP1074243 A2.

### Detailed description of the invention

According to an embodiment, the perfluoropolyether phosphate used within the scope of the present invention is the compound obtained by the reaction of ortho-phosphoric acid (alternatively phosphoric anhydride or phosphorus oxytrichloride can be used), with structure: O = P(OH)₃ with α-ϕ perfluoropolyether dihydric alcohol ethoxylate, a compound with a linear polymer structure having two hydroxyls in the terminal positions joined with a PFPE chain of Z type by a bridge constituted by one/two ethoxylenic units, and structure:

HO-(CH₂CH₂O)ₙ-CH₂-R_{f}-CH₂-(OCH₂CH₂)ₙ-OH

where R_{f}, n, p/q have been described previously.

According to an embodiment, the chain of PFPE (R_{f}), linear and symmetrical (Z type), with as main repeating groups (CF₂CF₂O) and (CF₂O) in random distribution, is obtained by photo-polymerization of tetrafluoroethylene in presence of oxygen. Typically, from a structural point of view, this is a copolymer chain, although it originates from a single monomer, which is partially degraded with a radical mechanism, that involves oxygen and is activated by UV radiation.

In accordance with a preferred embodiment of the invention, the chain of PFPE (R_{f}) has a MW equal to about 1500.

The -OH groups of ortho-phosphoric acid can condense with the hydroxyl groups of the alcohol to form phosphate esters. Since ortho-phosphoric acid has three -OH groups it can esterify with one, two or three alcohol molecules to form a mono-, di- or tri-esters. The reaction is typically carried out under ortho-phosphoric acid deficiency conditions with respect to dihydric alcohol, so as to have a remarkable predominance of the monoester, together with smaller quantities of diester and absence of triester. Therefore, phosphoric acid esters are produced. Besides, the esterification reaction produces difunctional derivatives or diphosphates (mainly monoester diphosphate with minor amounts of diester of iphosphate) because of the presence of two hydroxyls in the terminal positions of the perfluoropolyether alcohol. For love of simplicity, these esters are named PFPE phosphate and only the monoester diphosphate is reported here-under:

(HO)₂OP-O-(CH₂CH₂O)ₙ-CH₂-R_{f}-CH₂-(OCH₂CH₂)ₙ-O-P-O(OH)₂

To a chain of PFPE (R_{f}) with molecular weight of 1500 corresponds a PFPE phosphate with molecular weight of 2500, due to the contributions to the mean value of the phosphate groups, of the oxyethylenic groups and of the diester (where two chains PFPE are bonded to the same phosphate group). This PFPE phosphate is available in the market as Fomblin HC/P2-1000 (INCI name: "Polyperfluoroethoxymethoxy difluoroethyl PEG phosphate) from Solvay Solexis, Milan.

Typically, Fomblin® HC/P2-1000 is a viscous liquid, of straw-yellow to brown color, available on the market as phosphoric ester in acid form, completely insoluble in water and in oils, but soluble in polar solvents.

Within the scope of the invention, the continuous phase of the obtained HIPE emulsions is an aqueous phase, which possibly contains a polar solvent, and in which it is possible to solubilise PFPE phosphate, the dispersed or internal phase is an oil or a mixture of oils, optionally containing one or more active substances.

Typically, in the method of the invention, the aqueous solution of PFPE phosphate is obtained with or without the use of a polar solvent.

The modes for dissolving in water PFPE phosphate, whose solubility is, for its structure, more influenced by the two very polar terminals than by the molecular weight and the type of the PFPE chain, produce aqueous phases, distinguishing by the pH, typically from very acid to neutral, and by the possible presence of a polar solvent, having the concentration of few percentage units.

According to an embodiment, PFPE phosphate can be dissolved in water for partial or total neutralization with a base. Generally, an alkali is used, typically sodium hydroxide in water, or an organic base. The neutralizing agent is added gradually to the aqueous dispersion of PFPE phosphate, preferably under heating, for example in a temperature range of 60-90°C, and under stirring. Transparent solutions are obtained, with pH comprised between 4 and 12, preferably 5 to 7, and concentrations of the PFPE phosphate in the range of 5-20%, to be diluted, if necessary, by simple addition of water, to reach more suitable concentrations, even very low, as 0.1%, or even lower.

According to another embodiment, it is possible to obtain aqueous solutions of PFPE phosphate without the use of neutralizing agents, by preparing solutions, for example up to 40-50%, in polar solvents, to be diluted adding gradually water, that becomes the main component of these solutions, with concentrations of PFPE phosphate typically in the range of 5-20%, for which further dilutions with water are possible, to reach more suitable concentrations, even very low, as 0.1% or even lower.

Typically, the pH of these solutions can vary from about 1.5 up to 7, due to the possibility of adding a neutralizing agent. According to one embodiment, the solvent now used can be a volatile solvent, chosen for example from among ethanol, propanol, isopropyl alcohol, acetone, methylal; or a glycol, for example ethylene glycol, propylene glycol, 1,4-butanediol, 1,2-pentanediol, 1,6-hexanediol, dipropylene glycol; or a glycol ether, for example diethylene glycol monoethyl ether, dipropylene glycol monoethyl ether. It is preferred to use ethanol, isopropyl alcohol, propylene glycol and 1,2-pentanediol. In these conditions of hydroalcoholic or hydroglycolic environment with great prevalence of water, any type of oil is insoluble.

According to a further embodiment, the solubilization in water is obtained at high pressure. For example, acting with high pressure homogenizers (Panda® model of Niro-Soavi, Parma), it is possible to obtain a limited solubilization of PFPE phosphate in water, without neutralization or use of a solvent. In these conditions, the oil remains insoluble.

According to an aspect of the invention, an HIPE emulsion is obtained directly.

In accordance with this further aspect, an oil is added to the aqueous solution of PFPE phosphate, acting preferably with phases being heated under stirring until a fluid emulsion is obtained. Further oil is added to this fluid emulsion, suitably keeping up the stirring, until a viscous emulsion is obtained, having a micellar structure, visible under a microscope. The obtained viscous emulsion is an HIPE emulsion, that shows oil repellence in the filter paper test.

In accordance with an embodiment, the method for obtaining directly an HIPE emulsion includes a first addition of an oil, for example a cosmetic oil, from one tenth to one third of the total quantity, to the aqueous solution of PFPE phosphate, with pH between 1 and 14, preferably between 3 and 8, and still more preferably between 4 and 7, acting with the two phases being pre-heated on plate at 70-80°C (when there are no contraindications caused by the thermal instability or volatility of some ingredients), with strong mechanical stirring (for example with a Silverson L5M at 5000 revolutions/minute) for a few minutes.

A fluid emulsion is obtained, containing preferably between 10 and 50 parts of oil per 100 parts of the aqueous phase, and still more preferably, 20-30 parts of oil per 100 parts of the aqueous phase. Further oil is added gradually to this fluid emulsion, keeping up the homogenizer stirring and maintaining the temperature in the range of 75-80°C, until the proportion of 3 parts of oil per one part of the aqueous phase is obtained (and, if necessary, exceeded).

The stirring is maintained typically for 3-20 minutes, for example for about 10 minutes. It is let cooled at room temperature maintaining a fight stirring: a viscous emulsion is obtained, that shows a micellar structure under a microscope (100 magnifications) and oil repellence in the filter paper test. In the obtained emulsion, PFPE phosphate is typically in a proportion between 0.1 and 100 parts, preferably between 0.5 and 5 parts, still more preferably between 1 and 2 parts per 100 parts of oil. The oil is typically in a proportion between 3 and 15 parts, preferably between 3 and 10 parts, and still more preferably between 3 and 5 parts per one part of the aqueous phase (with a solvent, if necessary).

According to another aspect of the invention, the HIPE emulsion is obtained through centrifugation of superfluid emulsions.

According to this aspect, the method includes the dispersion of an oil in an aqueous solution of PFPE phosphate, up to the formation of a superfluid emulsion with viscosity lower than 10 mPa/s, dispersible in water.

The obtained superfluid emulsion is then concentrated, for example by centrifugation, obtaining a viscous emulsion, distinct from the aqueous solution, or by separation of water due to evaporation (for example, with the use of a rotating evaporator).

The obtained viscous emulsion can be easily (re)dispersed in water, obtaining a fluid emulsion, from which the viscous emulsion can be obtained again by centrifugation, showing a reversible behaviour, which is not characteristic of the prior art emulsions. The obtained emulsion shows a regular micellar structure under an optical microscope and demonstrates to be oil repellent in the filter paper test.

According to an embodiment, the method for obtaining a HIPE emulsion through superfluid emulsions includes the addition of an oil, preheated, for example, to 80°C, to an aqueous solution of PFPE phosphate (neutral or acid), pre-heated typically up to 70-80°C, with pH in the range of 1 to 14, preferably 3 to 8, and still more preferably 4 to 7, with strong stirring, for example for 10-20 minutes (Silverson L5M), at 5000 revolutions/minute. It is let cooled at room temperature maintaining a slight stirring: a very fluid emulsion is formed, that can be easily dispersed in water and looks like milk.

In the obtained emulsion, PFPE phosphate is typically in a proportion between 0.1 and 100 parts, preferably between 1 and 10 parts, still more preferably between 2 and 5 parts per 100 parts of oil. The oil is typically in a proportion between 0.1 and 200 parts per 100 parts of the aqueous phase (containing, if necessary, a minor amount of solvent), preferably in the range of 1 to 100 parts per 100 parts of the aqueous phase, and still more preferably 20 to 50 parts per 100 parts of the aqueous phase.

The concentration of the emulsion can be obtained by centrifugation, separating, with stratification at the bottom or at the top, a white mass, apparently homogeneous, that looks like a viscous emulsion (semi-solid), well distinguished from the remaining aqueous solution, almost transparent. It is possible to assess the content of water in the viscous emulsion (generally in the range of 10 to 25%) by weighing the amount of separated water. As an alternative to the centrifugation, the concentration can be carried out with other means, for example, a rotating evaporator.

It has been noted that, by means of the treatment with PFPE phosphate according to an aspect of the invention, not only does an emollient oil or a mixture of emollient oils acquire lipophobic and homophobic characters, but also improves the lubricity and sensory properties, which is advantageous, for example, in the formulation of skin moisturizing and protecting products, in particular against irritating liposoluble substances; in shaving products (lubricating action); in sun products (for increasing water resistance); in hair products (for more brilliance, anti-dirt/anti-smog effects and -conditioning action without build-up); in make-up products (for easier spreading and increased persistence).

It has also been observed that in case of other oils, such as chemical UV filters, potentially toxic if absorbed or like fragrances which are risky due to the presence of allergens, the lipophobicity of the emulsions and modified oils of the invention reduces or avoids the skin penetration, improving also the functionality of these ingredients due to the possibility of more homogenous distribution on the skin (UV filters) or a delayed or prolonged release (fragrances). Furthermore, lipophobicity, combined also with homophobicity leads to the non miscibility of oils or mixtures of oils treated separately with PFPE phosphate. Furthermore, PFPE phosphate imparts to oils the anti-sticking properties, typical of high fluorine content substances, improving the sensory properties of the finished products. Finally, the emulsions and modified oils of the invention have enough hydrophylicity that allows the dispersion in water without emulsifying agents.

According to an embodiment, an emollient-based HIPE emulsion can be used as a finished product for its lubricating and protective properties.

According to another embodiment, the emulsion can also act as exfoliating agent, with acid and neutral pH, in case of concentrations of PFPE phosphate (1-5%) higher than those required for emulsification.

According to a further embodiment, the HIPE emulsions of the invention are suitable for carrying active principles, for example pharmacologically active substances. The lipophobic character of the oil/emulsion is advantageous for avoiding skin absorption of active substances, like UV filters, potentially risky ingredients in case of absorption depending on their toxicity, and of fragrances, risky ingredients due to the possible presence of allergens. In addition to the safety advantages (reduced or no release for ingredients with toxicity risks), there is the possibility, with these two typologies of active substances, of influencing their performance:
- in the case of UV filters (sunscreens), with a more uniform application on the skin (or on the hair);
- in the case of fragrances, by modifying the volatility profile with the advantage of a longer persistence.

According to another embodiment, the method of the invention presents the following advantages from the formulation point of view:
- the addition of an ingredient or a component to a formulation in the form of an oil or an emulsion or in a gel, operating in mild conditions (at room temperature and with a minimum stirring) while ensuring in any case an accurate and homogenous dispersion;
- obtaining polyphasic systems, by varying the procedures, in such a way as to obtain a new type of multiple emulsions with oily phases separated and dispersed in the same aqueous phase (O₁ + O₂ + O₃.....)/W;
- obtaining a "delivery" effect with active agents, for which the skin absorption is advantageous, simply incorporating them in an oily phase non-treated with PFPE phosphate combined with "no delivery" effect for the risky agents, as previously said;
- preparing fluid emulsions (suitable for the impregnation of tissues and for spray systems) by simple dilution with water.

Within the scope of the invention, the term oil means a liquid that is insoluble in water.

Within the scope of the method of the invention it is possible to use any oil or even mixtures of different oils, as well as the solutions and the dispersions of solids in an oil.

In particular, emollients and other oils for cosmetic use, as well as oils containing fragrances, can be used.

According to an embodiment, within the scope of the invention one or more oils chosen from the following are used:
- hydrocarbons (mineral oils, paraffins and isoparaffins, low molecular weight polyolefins, squalane, linear and cyclical terpenes);
- long chain alcohols (ethylhexyl dodecanol, hexyldecanol, isostearilic alcohol, cetearilic alcohol);
- fatty acid ethers (dicaprylic ether);
- fatty acid ethers with propylene glycol (PPG-11 stearyl ether, PPG-11 stearyl ether);
- monocarboxylic fatty acid esters with synthetic alcohols (ethylhexyl palmitate, isopropyl palmitate, isopropyl myristate, isopropyl Isostearate, hexyl laurate);
- esters of synthetic monocarboxylic acids with fatty alcohols (C₁₂-₁₅ alkyl benzoate, cetyl/stearyl isononanoate);
- monocarboxylic fatty acid esters with fatty alcohols (tridecyl stearate, stearyl ricinoleate);
- esters of synthetic monocarboxylic acids with synthetic alcohols (ethylhexyl octanoate);
- esters of synthetic dicarboxylic acids with synthetic alcohols (diisopropyl adipate, dibutyl adipate, diisopropyl sebacate, dibutyl sebacate);
- esters of synthetic carboxylic acids with fatty alcohols (myristyl adipate);
- esters of monocarboxylic acids with propoxylated glycols (PEG-4 diheptanoate);
- fatty acid esters with polyhydric alcohols (glycerol tricaprylate/caprate, pentaerythritol tetracaprylate/caprate, pentaerythritol tetraoleate, sucrose alkyl esters);
- esters of hydroxy acids with fatty alcohols (tridecyl salicylate, myristyl tartrate, myristyl lactate, tri-C₁₂₋₁₃ alkyl citrate),
- esters of polycarboxylic acids with fatty alcohols (trimethylated tridecyl);
- vegetable oils (avocado oil, macadamia oil, castor oil, sesame oil, almond oil, wheat germ oil, jojoba oil, sunflower seed oil);
- hydrogenated vegetable oils,
- unsaponifiable fractions of vegetable oils,
- vegetable butters (cacao butter, shea butter);
- animal oils (lanolin oil);
- waxes (liquids, semi-solids and low-melting solids)

According to a further embodiment, also oil-based products can be used for producing a HIPE emulsion according to an aspect of the invention, chosen from:
- chemical UV filters in the form of liquids, such as ethylhexyl methoxycinnamate (Parsol MCX of DSM, Holland) or octocrylene (Uvinul A539 of BASF, Germany) or also in the form of solids soluble in liquid filters or in emollient oils, such as butyl methoxydibenzoylmethane (Parsol 1789 of DSM);
- essential oils and fragrances (synthetic or natural and their combinations) as such or in solution in emollient oils.

### Oil repellence

The emulsions obtained with the method of the invention are HIPE emulsions that show oil repellence proved by a very simple test, suitable for ready evaluation of the oil repellence of a preparation in the form of oil or of a preparation containing an oil.

About 0.5 ml of this product is applied on a surface of 10 x 10 cm of filter paper (Albet SA, Barcellona, Spain), without additives and with weight of 600 mg/100 cm², acting so as to have a uniform layer. After 20 minutes, when the evaporation of water and possible volatile solvents has been completed, a drop of mineral oil is applied on the treated area. Another area, non-treated or treated with another preparation, is used for comparison. Then, the form, the transparency and above all the absorption time are observed. Obviously, the test has only indicative value, but it allows to define as "oil repellent" (lipophobic) an oil or a preparation with an absorption time higher than 30 minutes, to be compared with the almost immediate absorption present in the non-treated area. The test allows also to evaluate the capacity of a "modified oil" to maintain itself as it is, since it is a component of an emulsion.

### Content of water.

The content of water in the HIPE emulsions has been measured experimentally by an independent research laboratory (Farcos, Milan), with measures done before and after drying in stove, up to a constant weight.

### Apparatuses and instrumentation

The following laboratory apparatuses and instrumentation have been used:
- homogenizer Silverson L5M, Silverson Machines, Ltd., Waterside, Chesham (United Kingdom), with standard heads, stator and molecular sieve and in-line head;
- high pressure homogenizer Panda 2k, Niro Soavi SpA, Parma (Italy);
- magnetic stirrers with heating plates;
- centrifuge Biofuge 17RS with heat regulation, Heraeus Sepatech GmbH, Osterode/Harz (Germany);
- rotating evaporator Rotavapor R-114, Büchi Labortechnik AG, Flawil (Switzerland), with a temperature bath (Büchi Waterbath B-480) and a water-cooled condenser;
- microscope DM 2000, Leica Microsystems Heidelberg GmbH (Germany), with a Leica DFC 290 digital camera and a Leica LAS software, a Pentium-43 and LCD TFT processor, and a 19" monitor;
- pH meter Metrohom, with combined glass electrode and temperature control probe, Metrohm AG, Herisau (Switzerland);
- viscometer Brookfield DV-I, spindle set of the Brookfield Engineering, Laboratories Inc., Middleboro, MA (USA);
- thermometers.

The present invention claims the priority of the Italian application No. MI2008A1339 to the same Applicant whose content is incorporated by reference and hereby fully integrated.

The following examples are provided as a mere illustration of the present invention and must not be meant as limiting the protection scope as it results from the enclosed claims.

### Example 1 - Superfluid emulsion (mineral oil/neutral PFPE phosphate) to obtain HIPE by centrifugation

Separately, an aqueous solution of PFPE phosphate, available on the market as phosphoric acid ester, insoluble in water (Fomblin®HC/P2-1000, produced by Solvay Solexis SpA, Milan) is prepared. This solubilisation is to be carried out very carefully, with gradual additions, so as to avoid a pH shock, of an aqueous solution of sodium hydroxide to an aqueous dispersion of acid PFPE phosphate heated to 80°C, under magnetic stirring, as follows:
- Aqueous solution

| | |
|---|---|
| Sodium hydroxide | 0.76 parts |
| Demineralized water | 8.00 parts |

- Aqueous dispersion

| | |
|---|---|
| PFPE phosphate (Fomblin HC/P2 1000) | 20.00 parts |
| Demineralized water | 71.24 parts |
| *Total* | *100.00 parts (containing 20 parts by weight of PFPE phosphate)* |

A perfectly transparent solution of PFPE phosphate (20%) is obtained, with pH comprised in the range of 5 to 7, which can be diluted with water to the most suitable concentration.

The emulsification consists of the addition of mineral oil (BFR070, Paraffinum Liquidum FU, ACEF SpA, Piacenza, Italy), heated to 80°C, step (a) of the emulsion process, to a diluted solution of neutral PFPE phosphate in water heated to 80°C, step (b) of the emulsion process, under the stirring with Silverson L5M (5000 revolutions/min), for 10 minutes, then it is let cooled down to room temperature under slight stirring:

| | (% by weight) |
|---|---|
| a) Mineral oil (BFR070 Paraffinum Liquidum FU) | 30.0 |
| b)Neutral PFPE phosphate (20% in water) | 5.0 |
| Demineralized water | 65.0 |
| *Total* | *100.0* |

A superfluid emulsion (viscosity < 10mPas/s) is obtained, which looks like white milk, easily dispersible in water. The centrifugation (treatment for 20 minutes, 5000 revolutions/min and 25°C) results in stratification without separation of oil, with the upper layer that looks like a "viscous" white emulsion, while the underlying aqueous part, with the pH = 7.3 has a slightly translucent look. This viscous emulsion, that can be dispersed in water by a simple manual stirring, is stable to other centrifugation treatments and "oil repellent" in the filter paper test and shows a fine and homogenous structure of micellar type under optical microscope.

During the emulsification, the necessary compensation of evaporated water was done, so as to make it possible to calculate, although in approximate way, the content of water of the viscous emulsion obtained by centrifugation. From the weight of the water separated by centrifugation, it was possible to calculate the percentage by weight of water and of oil in the viscous emulsion, that are equal to 20.6 and 79.4% by weight, respectively.

These values are in accordance with those obtained by analytical measures of an independent laboratory (loss due to drying, at 105°C, up to a constant weight), which report a content of water equal to 24.7%, with the rest constituted by oil (and PFPE phosphate) for the 75.2% by weight, corresponding to the 77.9% by volume (density of mineral oil 0.86 g/ml), a percentage higher than the critical level of the 74%, therefore it is within the HIPE emulsions field.

### Example 2 - HIPE direct emulsion (mineral oil/neutral PFPE phosphate)

Actions are as in example 1, for the preparation of a solution of neutral PFPE phosphate in water (20%), to be diluted with water, then producing directly an HIPE emulsion of mineral oil, with the following proportions:

| | (% by weight) |
|---|---|
| a) Mineral oil (BFR070 Paraffinum Liquidum FU) | 75.0 |
| b) Neutral PFPE phosphate (20% in water) | 5.0 |
| Demineralized water | 20.0 |
| *Total* | *100.0* |

The mineral oil (a) and the aqueous solution of PFPE phosphate (b) are heated on a plate to 80°C. A portion (about one tenth) of the mineral oil is added to the aqueous solution under stirring (Silverson L5M) for 10 minutes, at 5000 revolutions/minute. A white, very fluid, dispersible in water, emulsion is formed. The remaining part of oil is added slowly, maintaining this milk at 80°C while stirring. A remarkable increase of viscosity is noted, then the stirring is continued for other 10 minutes. The emulsion is then cooled under slight stirring with formation of a viscous (semi-solid) emulsion, dispersible in water. The emulsion showed a minimum separation of water at the first centrifugation, while no water separation was observed after further centrifugations. The emulsion proved to be "oil repellent" in the filter paper test, showed a structure very similar to that of example 1 under the optical microscope and did not show separation of water or of oil by centrifugation (5000 revolutions/minute, 20 minutes, at 25°C).

During the emulsification, the evaporated water has been compensated, so as to maintain approximately constant the percentages by weight of the aqueous phase and of the oily phase; 25% and 75%, respectively. The analytical result (loss due to drying at 90°C, up to the constant weight) has given a content of water of 21.8%, from which it results that the oily phase is equal to 78.2%. With these values, taking into consideration the density of the mineral oil (0.86g/ml), percentages by volume of 19.3% and 80.7%, respectively, are obtained.

### Example 3 - HIPE direct emulsion (mineral oil/neutral PFPE phosphate/glycerol): comparison with example 2

Example 2 is repeated, substituting a part of water with the glycerol, as reported in the literature of the Mitsubishi-Kagaku Foods Corp.

| | (% by weight) |
|---|---|
| a) Mineral oil (BFR070 Paraffinum Liquidum FU) | 75.0 |
| b) neutral PFPE phosphate (20% in water) | 5.0 |
| c) Vegetal glycerol FU-Ph Eur. | 10.0 |
| Demineralized water | 10.0 |
| *Total* | *100.0* |

A translucent "emulsion" is obtained, which, due to the centrifuge action, shows a consistent oil separation, with phases stratification.

### Example 4 - Superfluid emulsion (mineral oil/acid PFPE phosphate) to obtain HIPE by centrifugation.

A concentrated alcoholic solution of acid PFPE phosphate (20% by weight) is prepared separately, solubilising directly Fomblin® HC/P2-1000 in ethanol (98%) under magnetic stirring for 20 minutes. The obtained solution, slightly opalescent, becomes perfectly transparent after a gradual addition of little hot water (approximately 5 parts per 100 parts of PFPE phosphate in alcohol). This concentrated alcoholic solution (20%) is diluted with other water, down to 1%. The pH is measured: 2.7.

A superfluid emulsion is obtained as in example 1, with the only substitution of the neutral PFPE phosphate with the acid PFPE phosphate:

| | (% by weight) |
|---|---|
| a) Mineral oil (BFR070 Paraffinum Liquidum FU) | 30.0 |
| b) Acid PFPE phosphate (20% in ethanol) | 5.0 |
| Demineralized water | 65.0 |
| *Total* | *100.0* |

A superfluid emulsion is obtained, easily dispersible in water, with pH = 3.2, from which a viscous emulsion is separated by centrifugation (20 minutes, 5000 revolutions/minute, 25°C). This viscous emulsion has characteristics (dispersibility in water, oil repellence, observations under optical microscope) similar to the emulsion of example 1.

### Example 5 - HIPE direct emulsion (mineral oil/acid PFPE phosphate)

Actions are performed as in example 2, with the only substitution of neutral PFPE phosphate with acid PFPE phosphate:

| | (% by weight) |
|---|---|
| a) Mineral oil (BFR070 Paraffinum Liquidum FU) | 75.0 |
| b) Acid PFPE phosphate (20% in ethanol) | 5.0 |
| Demineralized water | 20.0 |
| *Total* | *100.0* |

A viscous emulsion is obtained, having characteristics (dispersibility in water, oil repellence and observations under optical microscope) similar to those of the emulsion of example 2.

### Example 6 - Superfluid emulsion (ethylhexyl palmitate/neutral PFPE phosphate) and centrifugation to HIPE

Actions are performed as in example 1, substituting mineral oil with ethylhexyl palmitate (Cegesoft C 24, Cognis, Germany), as follows:

| | (% by weight) |
|---|---|
| a) Ethylhexyl palmitate (Cegesoft C24) | 30.0 |
| b) Neutral PFPE phosphate (20% in water) | 5.0 |
| Demineralized water | 65.0 |
| *Total* | *100.0* |

A superfluid emulsion is obtained, dispersible in water, from which a viscous emulsion is obtained by centrifugation, said viscous emulsion having characteristics (dispersibility in water, oil repellence, micellar structure) similar to those of example 1. The viscous emulsion is mixed with 20% of ethylhexyl palmitate, non emulsified, dyed with red color (D&C N°17KT007 of the LCW/Sensient, France/USA) and is stirred manually. After two-day rest period, the centrifugation is performed, obtaining the formation of two layers: only the upper one (not emulsified) appears colored.

### Example 7 - HIPE direct emulsion (ethylhexyl palmitate/neutral PFPE phosphate)

Actions are performed as in example 2 in the direct preparation of a HIPE emulsion of ethylhexyl palmitate (Cegesoft C24, Cognis, Germany), with neutral PFPE phosphate, in the following proportions:

| | (% by weight) |
|---|---|
| a) Ethylhexyl palmitate (Cegesoft C24) | 75.0 |
| b) Neutral PFPE phosphate (20% in water) | 5.0 |
| Demineralized water | 20.0 |
| *Total* | *100.0* |

After a two step addition of oil, a very viscous emulsion is obtained and let cooled down to room temperature, under slight stirring. As far as characteristics are concerned (dispersibility in water, oil repellence and observations under optical microscope), this viscous emulsion is similar to those of examples 2 and 5.

### Example 8 - Direct HIPE emulsion of emollients

Actions are performed as in example 2, preparing a direct HIPE emulsion of emollients: isostearyl isostearate (isostearyl ilsostearate, Gattefosse S.A., France), caprylic/capric triglyceride (Myritol 318, Cognis, Germany), butyl cocoate (Cocoate BG, Gattefossé S.A., France), adding an aqueous solution of PFPE phosphate to the mixture of emollients:

| | (% by weight) |
|---|---|
| a) Isostearyl isostearate | 22.5 |
| Butyl cocoate | 22.5 |
| Caprylic/capric triglyceride | 30.0 |
| b) PFPE phosphate (20% in water) | 5.0 |
| Demineralized water | 20.0 |
| *Total* | *100.0* |

A white viscous emulsion is obtained, which does not show separation of oil by centrifugation, while a very modest sedimentation separation of slightly opalescent water is obtained. This aqueous part is separated and the centrifugation of the viscous emulsion is repeated, without further separation.

### Example 9 - Superfluid emulsion (volatile silicone/neutral PFPE phosphate) to obtain a HIPE by centrifugation

Actions are performed as in example 1, substituting the mineral oil with a volatile silicone, chemically a cyclopentasiloxane (Baysilone SF, GE Silicones, Germany/USA), and addition of an aqueous solution of PFPE phosphate at 60°C to the volatile silicone heated at about the same temperature.

| | (% by weight) |
|---|---|
| a) volatile silicone (Baysilone SF 1202) | 30.0 |
| b) neutral PFPE phosphate (20% in water) | 5.0 |
| Demineralized water | 65.0 |
| *Total* | *100.0* |

An aqueous phase, with pH 7.6, and a viscous emulsion are separated, due to centrifugation, from a superfluid emulsion, easily dispersible in water. This viscous emulsion is stable after a further centrifugation treatment, but after a few days on the shelf, it forms a liquid transparent layer, wholly similar to the non-treated silicone.

### Example 10 - Superfluid emulsion (ethylhexyl methoxycinnamate/neutral PFPE phosphate) to obtain HIPE by centrifugation

Actions are performed as in example 1, substituting the mineral oil with ethylhexyl methoxycinnamate (Parsol MCX, DSM, Holland):

| | (% by weight) |
|---|---|
| a) Ethylhexyl methoxycinnamate (Parsol MCX) | 30.0 |
| b) Neutral PFPE phosphate (in water at 20%) | 5.0 |
| Demineralized water | 65.0 |
| *Total* | *100.0* |

The addition of the ethylhexyl methoxycinnamate makes immediately the solution turbid, forming the emulsion; the stirring is maintained for 10 minutes at 5000 revolutions/minute, then the solution is let cooled under a slight stirring. A superfluid emulsion, easily dispersible in water, is obtained. The centrifugation (20 minutes, 5000 revolutions/minute, 25°C) separates a white viscous emulsion, dispersible in water, while it is not possible to disperse it in the same ethylhexyl methoxycinnamate without magnetic stirring. This viscous emulsion appears oil repellent in the filter paper test, with resistance to the absorption of the mineral oil and of the same ethylhexyl methoxycinnamate, while it shows a micellar homogenous structure under optical microscope.

### Example 11 - Conventional emulsion (ethylhexyl methoxycinnamate/potassium cetyl phosphate): comparison with example 10

Example 10 is repeated, substituting the PFPE phosphate with potassium cetyl phosphate (Amphisol K, DSM, Holland) as powder dispersed in water at 100%, acting with the following percentage proportions.

| | (% by weight) |
|---|---|
| a) Ethylhexyl methoxycinnamate (Parsol MCX) | 30.0 |
| b) Potassium cetyl phosphate (Amphisol K) | 5.0 |
| Demineralized water | 65.0 |
| *Total* | *100.0* |

A fluid emulsion is obtained, which gelatinizes when cooled. This preparation disperses with simple manual stirring in ethylhexyl methoxycinnamate and is destructured in water under stirring with Silverson L5M. In the filter paper test, it does not show resistance to the absorption of mineral oil and of ethylhexyl methoxycinnamate.

### Example 12 - Superfluid emulsion (ethylhexyl methoxycinnamate/acid PFPE phosphate) to obtain HIPE by centrifugation

Actions are performed as in example 3, substituting the mineral oil with ethylhexyl methoxycinnamate and carrying out emulsification with acid PFPE phosphate, with the following percentage proportions:

| | (% by weight) |
|---|---|
| a) Ethylhexyl methoxycinnamate (Parsol MCX) | 30.0 |
| b) Acid PFPE phosphate (in 20% ethanol) | 5.0 |
| Demineralized water | 65.0 |
| *Total* | *100.0* |

A viscous emulsion, with characteristics similar to those of the emulsions of examples 1 and 10, is obtained by centrifugation of the superfluid emulsion.

### Example 13 - Combination of two superfluid emulsions, with separation of two HIPE by centrifugation

The emulsification with neutral PFPE phosphate of two oils (colored by the addition of a different dye), ethylhexyl methoxycinnamate (A) and ethylhexyl palmitate (B) is carried out separately, acting according to the procedure of example 1 and the following percentage proportions:

**A) Emulsion of ethylhexyl methoxycinnamate:**

| | (% by weight) |
|---|---|
| a) Ethylhexyl methoxycinnamate (Parsol MCX) | 49.98 |
| Dye (D&C Red N°17K7007, CLW) | 0.02 |
| b) Neutral PFPE phosphate (20% in water) | 10.00 |
| Water | 40.00 |
| *Total* | *100.00* |

**B) Emulsion of ethylhexyl palmitate:**

| | (% by weight) |
|---|---|
| a) Ethylhexyl palmitate (Cegesoft C24) | 49.98 |
| Dye (D&C Vert N°11, CLW) | 0.02 |
| b) Neutral PFPE phosphate (20% in water) | 10.00 |
| Demineralized water | 40.00 |
| *Total* | *100.00* |

The two superfluid emulsions are pink (A) and light blue (B). Equal parts of these two emulsions are mixed with manual stirring, obtaining a fluid beige emulsion. Centrifugation is performed (20 minutes, 5000 revolutions/minute, 25°C) and stratification is obtained with the upper light blue layer separated by a pink layer and with an aqueous layer at the bottom.

### Example 14 - Superfluid emulsion (solid filter dissolved in a liquid filter/neutral PFPE phosphate) to obtain HIPE by centrifugation

Example 1 is repeated, substituting the mineral oil with an oily solution constituted by a solid solar filter (Parsol 1789, DSM, Holland) dissolved in a liquid filter (Parsol MCX, DSM Holland), with the following proportions:

| | (% by weight) |
|---|---|
| a) INCI name (commercial name) | |
| Butyl methoxydibenzoyl methane (Parsol 1789) | 9.0 |
| Ethyl methoxycinnamate (Parsol MCX) | 21.0 |
| b) Neutral PFPE phosphate (20% water) | 10.0 |
| Demineralized water | 60.0 |
| *Total* | *100.0* |

A superfluid emulsion is obtained, which left to rest for a couple of days does not show stratification; therefore it is centrifuged (20 minutes, 5000 revolutions/minute, 25°C), and the upper aqueous layer of translucent look is settled, with separation from a white viscous emulsion. This emulsion is dispersed in water (manual and magnetic stirring) obtaining a milk, which becomes stratified by centrifugation with formation of a white viscous emulsion, seemingly not altered. Both the viscous emulsion and the aqueous solution appear to be "oil-repellent" in the filter paper test. The dispersion in water with subsequent centrifugation is repeated again, obtaining a viscous "oil repellent" emulsion, while the aqueous solution reveals to be "not oil repellent".

### Example 15 - Superfluid emulsion (mixture of UV filters/neutral PFPE phosphate) to obtain HIPE by centrifugation

Example 1 is repeated, substituting the mineral oil with a solution of solid filters in liquid filters, acting with the following percentage proportions:

| | (% by weight) |
|---|---|
| a) INCI name (commercial name) | |
| Butyl methoxydibenzoyl methane (Parsol 1789, DSM) | 6.0 |
| Ethylhexyl methoxycinammate (Parsol MCX, DSM, Holland) | 16.0 |
| Diethylamino hydroxybenzoyl hexyl benzoate (Uvinul APlus, BASF) | 6.0 |
| Ethylhexyl triazone (Uvinul T150, BASF) | 8.0 |
| Octocrylene (Uvinul N539 T) | 2.0 |
| b) Neutral PFPE phosphate (20% in water) | 10.0 |
| Demineralized water | 52.0 |
| *Total* | *100.0* |

A superfluid emulsion is obtained, slightly dyed with yellow, which after the centrifugation (20 minutes, 5000 revolutions/minute, 25°C) produces allows to obtain a viscous yellow emulsion, without oil separation. This emulsion proves to be "oil repellent" and shows a fine and regular micellar structure under the optical microscope.

### Example 16 - HIPE direct emulsion (mixture of solar filters/neutral PFPE phosphate)

A HIPE emulsion is prepared with direct procedure (example 2) acting with the mixture of UV filters (solution of the solid filters in the liquid filters) and with neutral PFPE phosphate:

| | (% by weight) |
|---|---|
| a) INCI name (commercial name) | |
| Butyl methoxydibenzoyl methane (Parsol 1789, DSM) | 12.0 |
| Ethylhexyl methoxycinammate (Parsol MCX, DSM, Holland) | 30.0 |
| Diethylamino hydroxybenzoyl hexyl benzoate (Uvinul APlus, BASF) | 12.0 |
| Ethylhexyl triazone (Uvinul T150, BASF) | 12.0 |
| Octocrylene (Uvinul N539 T) | 9.0 |
| b) Neutral PFPE phosphate (20% water) | 5.0 |
| Demineralized water | 20.0 |
| *Total* | *100.0* |

A yellow viscous emulsion is obtained, which shows by centrifugation the separation of a small part of water as upper layer. This aqueous portion is separated and the centrifugation is repeated, without further water separation. A viscous emulsion is obtained with characteristics (appearance, observation under microscope and oil repellence) similar to those of the HIPE emulsion of example 15.

### Example 17 - Multiphase HIPE emulsions

Equal parts of two HIPEs, obtained with direct process according to example 8 (emollients) and example 16 (UV filters), are mixed by manual stirring. After centrifugation a stratification of the two viscous emulsions is noticed, with an upper layer constituted by a white emulsion and a lower layer by a yellow emulsion, without oil separation. The two viscous emulsions are re-mixed and homogenized with manual stirring, then carrying out a second centrifugation: the system is still stratified, without visible destructuring of the emulsions.

The two HIPEs are diluted separately, by adding three parts of water per each part of emulsion and the dispersions are homogenized with manual stirring, obtaining fluid emulsions. The two fluid emulsions are mixed and centrifugation is carried out: a formation of three layers is observed, constituted by a white emulsion (upper layer), an opalescent aqueous phase (intermediate layer) and by a yellow emulsion (lower layer).

### Example 18 - Superfluid emulsion (fragrance/neutral PFPE phosphate) to obtain HIPE by centrifugation

Example 1 is repeated, substituting the mineral oil with a fragrance (Code 748/M, GRC Parfum, Milan). Actions are performed without preheating of the two phases: a superfluid emulsion is obtained, without evidence of non-emulsified fragrance. After the centrifugation (60 minutes, 5000 revolutions/minute, 25°C), a stratification is obtained with a viscous emulsion in the upper layer.

### Example 19 - Diluted emulsion for tissues, by dilution of an HIPE emulsion

0.5 parts of the HIPE emulsion of example 18 (fragrance) and 4.5 parts of the HIPE emulsion of example 6 (emollient) are dispersed in 85 parts of an aqueous solution, in which 0.1 parts of xanthan gum have been pre-dispersed with manual stirring, followed by magnetic stirring for 20 minutes. A fluid emulsion is obtained with viscosity between 100 and 300 cps, suitable for tissue impregnation.

### Example 20 - Shaving cream with HIPE emulsion

A shaving cream is prepared by adding a mineral oil based HIPE emulsion (component b, composition of example 2) to a base (component a), at 30°C with manual stirring.

**Table 1 Shaving cream with two oily components, one of which is of HIPE type**

| Step | Ingredient | INCI name | Supplier | Content (% by weight) |
|---|---|---|---|---|
| a | Demineralized water | Aqua | | q.s. to 100 |
| | Vegetal glycerol | Glycerin | A.C.E.F. | 4.00 |
| | Rhodicare T | Xanthan gum | Rhodia (F) | 0.25 |
| | Phytosqualan | Phytosqualan | Sophin (F) | 10.00 |
| | Montanov 68 | Cetearyl glucoside, Cetearyl alcohol | Seppic (F) | 5.00 |
| | Optasense RM 50 | Sodium acrylate/sodium acryloyldimethyl taurate copolymer (and) Paraffinum liquidum (and) Trideceth-6 | Croda (UK) | 1.00 |
| | Perfume | Perfume | - | 0.20 |
| b | HIPE emulsion (example 2: mineral oil) | Aqua, Paraffinum liquidum, Polyperfluoroethoxymethoxy-difluoroethyl PEG phosphate, Sodium hydroxide | | 10.00 |
| | Total | | | 100.00 |

Characteristics of the finished product:
- Appearance: white emulsion,
- Viscosity: 100000 cps (Brookfield LVT), pH: 6.2.

### Example 21 - Roll-on deodorant comprising two HIPE emulsions

A roll-on deodorant is prepared with two components constituted by HIPE emulsions (examples 2 and 18) added to a base at 30°C with manual stirring.

**Table 2 Roll-on deodorant with two components constituted by HIPE emulsions**

| Step | Ingredient | INCI name | Supplier | Content (% by weight) |
|---|---|---|---|---|
| a | Demineralized water | Aqua | | q.s. 100 |
| | Vegetal glycerol | Glycerin | A.C.E.F. (I) | 4.00 |
| | Rhodicare T | Xanthan gum | Rhodia (F) | 0.10 |
| | Phytosqualan | Phytosqualan | Sophin (F) | 10.00 |
| | Montanov 202 | Arachidyl alcohol (and) Behenil alcohol (and) Arachidyl glucoside | Seppic (F) | 5.00 |
| | Fomblin HC/P2-1000 | Polyperfluoroethoxymethoxy difluoroethyl PEG phosphate | Solvay Solexis (I) | 1.00 |
| | Sodium hydroxide | Sodium hydroxide | Carlo Erba (I) | q.s. |
| b | HIPE emulsion (example 2: mineral oil | Aqua, Paraffinum liquidum, Polyperfluoroethoxy- methoxy difluoroethyl PEG phosphate, Sodium Hydroxide | | 10.00 |
| | HIPE emulsion (example 18: fragrance) | Aqua, Parfum, Polyperfluoroethoxymethoxy difluoroethyl PEG phosphate, sodium hydroxide | | 0.30 |
| | Total | | | 100.00 |

Characteristics of the finished product:
- Appearance: white emulsion
- Viscosity: 80000-100000 cps (Brookfield LVT)
- pH: 6.1

### Example 22 High protection sun cream comprising a HIPE emulsion with UV filters

A high protection sun cream is prepared with a component of the emulsion based on acrylic emulsifier, constituted by a HIPE emulsion with UV filters (example 16):

**Table 3 High protection sun cream (W/O emulsion) comprising a HIPE emulsion**

| Step | Descrizione | INCI | Supplier | Content (% by weight) |
|---|---|---|---|---|
| a | Demineralized water | Aqua | | 60.58 |
| | Cesesoft 24 | Ethylhexyl palmitate | Cognis (D) | 5.00 |
| | BFR070 Paraffinum liquidum FU | Paraffinum Liquidum | ACEF (I) | 5.00 |
| | Pemulen TR-1 | Acrylates/C10-30 alkylacrylate crosspolymer | Lubrizol (USA) | 0.30 |
| | Sodium hydroxide | Sodium hydroxide | Carlo Erba (I) | 0.12 |
| | Tinosorb S | Methylene bis-benzotri-azolyltetramethylbutylphenol | Ciba SC (CH) | 5.00 |
| b | HIPE emulsion based on solar filters (example 16) | Ethylhexyl methoxycinammate, Aqua, Butyl methoxydibenzoyl methane, Diethylamino hydroxy-benzoyl hexyl benzoate, Ethylhexyl triazone, Octocrylene, Polyperfluoroethoxymethoxy difluoroethyl PEG phosphate, Sodium hydroxide | | 24.00 |
| | Total | | | 100.00 |

Characteristics of the finished product:
- Appearance: white emulsion
- Viscosity: 80000-100000 cps (Brookfield LVT)
- pH = 6.5

## Claims

1. A HIPE oil in water (O/W) emulsion comprising a continuous aqueous phase and an oily internal phase, dispersed in the continuous aqueous phase, **characterized in that** the oily internal phase is greater than 74% by volume and said emulsion is homophobic and lipophobic and it comprises perfluoropolyether phosphate (PFPE) of the formula
**(HO)₂OP-O-(CH₂CH₂P)ₙ-CH₂-R_{f}-CH₂-(OCH₂CH₂)ₙ-O-P-O(OH)₂**
wherein:
n is from 1 to 2
R_{f} is a perfluoropolyether chain of formula:
-(CF₂CF₂O)ₚ-(CF₂O)_{q}-
with p/q from 0.5 to 3.0
the average molecular weight of R_{f} is comprised in the range of 500 to 4000,
preferably in the range of 1000 to 2000, more preferably 1400 to 1600 solubilised in the continuous aqueous phase.

2. A HIPE oil-in-water emulsion, as claimed in claim 1, **characterized in that** said perfluoropolyether phosphate is polyperfluoroethoxymethoxy difluoroethyl PEG phosphate (INCI name).

3. A HlPE oil in water emulsion, having an oily internal phase, greater than 74% by volume, lipophobic and homophobic, obtained by emulsification of an oily phase with a volume greater than 74% with an aqueous solution of perfluoropolyether phosphate (PFPE) of the formula:
**(HO)₂ OP-O-(CH₂CH₂O)ₙ-CH₂-R_{f}-CH₂-(OCH₂CH₂)ₙ-O-P-O(OH)₂**
wherein:
n from 1 to 2
R_{f} is a perfluoropolyether chain of formula:
-(CF₂CF₂O)ₚ-(CF₂O)_{q}-
with p/q from 0.5 to 3.0
the average molecular weight of R_{f}, is comprised between 500 and 4000, preferably from 1000 to 2000, more preferably from 1400 to 1600;
and by separation of the aqueous phase.

4. A HIPE oil-in-water, lipophobic and homophobic emulsion, as claimed in claim 3, **characterized in that** said perfluoropolyether phosphate is polyperfluoroethoxymethoxy difluoroethyl PEG phosphate.

5. A HIPE oil-in-water, lipophobic and homophobic emulsion, as claimed in claim 3 or 4, **characterized in that** said emulsification comprises:
dispersion under stirring of an oily phase in the aqueous solution of PFPE phosphate, up to formation of a fluid emulsion, and
concentration of the fluid emulsion by adding oil under stirring, up to formation of an oil repellent viscous emulsion.

6. A HIPE oil-in-water, lipophobic and homophobic emulsion, as claimed in claim 3 or 4, **characterized in that** said emulsification comprises:
dispersion under stirring of an oily phase in the aqueous solution of PFPE phosphate, up to formation of a superfluid diluted emulsion, and
concentration of the superfluid diluted emulsion by separation of the aqueous phase, up to formation of an oil repellent viscous emulsion.

7. A HIPE oil-in-water, lipophobic and homophobic emulsion, as claimed in any of the claims 3-6, **characterized in that** said oily phase includes an oil and one or more active substances dispersed therein.

8. A HIPE oil-in-water, lipophobic and homophobic emulsion, as claimed in claim 7, **characterized in that** said active substance is selected from solar filters, fragrances, pharmacologically active principles and mixtures thereof.

9. Cosmetic use of a HIPE oil-in-water, lipophobic and homophobic emulsion, as claimed in any of the claims 1-8, as a carrier of one or more active substances to reduce or to prevent the passage of said active substances through an external physiological barrier of a mammal's body.

10. Use of a HIPE oil-in-water, lipophobic and homophobic emulsion, as claimed in any of the claims 1-9 as a cosmetic for skin or hair.

11. A method for preparing a HIPE oil-in-water (O/W) emulsion, having an oily internal phase greater than 74% in volume, comprising the dispersion of a volume of an oil greater than 74% in an aqueous solution of perfluoropolyether phosphate (PFPE) of the formula
**(HO)₂OP-O-(CH₂CH₂O)ₙ-CH₂-Rf-CH₂-(OCH₂CH₂)ₙ-O-P-O(OH)₂**
wherein:
n from 1 to 2
R_{f} is a perfluoropolyether chain of formula:
-(CF₂CF₂O)ₚ-(CF₂O)_{q}-
with p/q from 0.5 to 3.0
the average molecular weight of Rf is in the range of 500 to 4000, preferably 1000 to 2000, more preferably 1400 to 1600, and wherein said perfluoropolyether phosphate is preferably polyperfluoroethoxymethoxy difluoroethyl PEG phosphate.

12. A method, as claimed in claim 11, **characterized in that** it comprises:
a dispersion step under stirring of an oily phase in the aqueous solution of PFPE phosphate, up to the formation of a fluid emulsion, and a concentration step including the addition of oil to the fluid emulsion under stirring, up to the formation of an oil repellent viscous emulsion.

13. A method as claimed in claim 12, **characterized in that** the dispersion step includes the addition of heated oil under stirring to the heated solution of PFPE phosphate, having a pH in the range of 3 to 8, up to obtaining a fluid emulsion, containing preferably from 10 to 50 parts of oil per 100 parts of the aqueous phase.

14. A method, as claimed in claim 12, **characterized in that** the concentration step includes:
addition of preheated oil under stirring to the fluid emulsion obtained in the dispersion step, up to reaching a proportion of 3 parts of oil per one part of aqueous phase, and maintaining of the stirring, up to obtaining an oil repellent viscous emulsion.

15. A method, as claimed in claim 11, **characterized in that** it includes:
a dispersion step under stirring of an oily phase in the aqueous solution of PFPE phosphate, up to formation of a superfluid diluted emulsion, and
a concentration step of the superfluid diluted emulsion by separation of the aqueous phase, preferably by centrifugation or by rotating evaporation, up to formation of an oil repellent viscous emulsion.

16. A method, as claimed in claim 15, **characterized in that** the dispersion step includes:
addition, under stirring, of the preheated oil to a preheated aqueous solution of PFPE phosphate, having pH in the range of 3 to 8, and
cooling down to room temperature, maintaining the stirring, up to the formation of a superfluid diluted emulsion.

## Patentansprüche

1. HIPE-Öl-in-Wasser-(O/W-)Emulsion, umfassend eine kontinuierliche wässrige Phase und eine ölige interne Phase, dispergiert in der kontinuierlichen wässrigen Phase, **dadurch gekennzeichnet, dass** die ölige interne Phase mehr als 74 Volumen-% ausmacht und die Emulsion homophob und lipophob ist und sie Perfluorpolyetherphosphat (PFPE) der Formel
**(HO)₂OP-O-(CH₂CH₂O)ₙ-CH₂-R_{f}-CH₂-(OCH₂CH₂)ₙ-O-P-O(OH)₂**
umfasst,
wobei:
n 1 bis 2 beträgt,
R_{f} eine Perfluorpolyetherkette der Formel:
-(CF₂CF₂O)ₚ-(CF₂O)_{q}-
mit p/q von 0,5 bis 3,0 ist,
das durchschnittliche Molekulargewicht von R_{f} im Bereich von 500 bis 4000, bevorzugt im Bereich von 1000 bis 2000, stärker bevorzugt 1400 bis 1600 liegt, aufgelöst in der kontinuierlichen wässrigen Phase.

2. HIPE-Öl-in-Wasser-Emulsion nach Anspruch 1, **dadurch gekennzeichnet, dass** das Perfluorpolyetherphosphat Polyperfluorethoxymethoxydifluorethyl-PEG-Phosphat (INCI-Name) ist.

3. HIPE-Öl-in-Wasser-Emulsion mit einer öligen internen Phase, die mehr als 74 Volumen-% ausmacht, und welche lipophob und homophob ist, erhalten durch Emulgierung einer öligen Phase mit einem Volumen, das mehr als 74 % ausmacht, mit einer wässrigen Lösung von Perfluorpolyetherphosphat (PFPE) der Formel:
**(HO)₂OP-O-(CH₂CH₂O)ₙ-CH₂-R_{f}-CH₂-(OCH₂CH₂)ₙ-O-P-O(OH)₂**
wobei:
n 1 bis 2 beträgt,
R_{f} eine Perfluorpolyetherkette der Formel:
-(CF₂CF₂O)ₚ-(CF₂O)_{q}-
mit p/q von 0,5 bis 3,0 ist,
das durchschnittliche Molekulargewicht von R_{f} zwischen 500 bis 4000, bevorzugt 1000 bis 2000, stärker bevorzugt 1400 bis 1600 liegt:
und durch Abtrennung der wässrigen Phase.

4. Lipophobe und homophobe HIPE-Öl-in-Wasser-Emulsion nach Anspruch 3, **dadurch gekennzeichnet, dass** das Perfluorpolyetherphosphat Polyperfluorethoxymethoxydifluorethyl-PEG-Phosphat ist.

5. Lipophobe und homophobe HIPE-Öl-in-Wasser-Emulsion nach Anspruch 3 oder 4, **dadurch gekennzeichnet, dass** die Emulgierung umfasst:
Dispersion unter Rühren einer öligen Phase in der wässrigen Lösung von PFPE-Phosphat bis zur Bildung einer flüssigen Emulsion, und
Einengung der flüssigen Emulsion durch Zugeben von Öl unter Rühren bis zur Bildung einer ölabweisenden viskosen Emulsion.

6. Lipophobe und homophobe HIPE-Öl-in-Wasser-Emulsion nach Anspruch 3 oder 4, **dadurch gekennzeichnet, dass** die Emulgierung umfasst:
Dispersion unter Rühren einer öligen Phase in der wässrigen Lösung von PFPE-Phosphat bis zur Bildung einer verdünnten suprafluiden Emulsion, und
Einengung der verdünnten suprafluiden Emulsion durch Abtrennung der wässrigen Phase bis zur Bildung einer ölabweisenden viskosen Emulsion.

7. Lipophobe und homophobe HIPE-Öl-in-Wasser-Emulsion nach einem der Ansprüche 3 bis 6, **dadurch gekennzeichnet, dass** die ölige Phase ein Öl und eine oder mehrere darin dispergierte aktive Substanzen umfasst.

8. Lipophobe und homophobe HIPE-Öl-in-Wasser-Emulsion nach Anspruch 7, **dadurch gekennzeichnet, dass** die aktive Substanz ausgewählt ist aus Sonnenfiltern, Duftstoffen, pharmakologisch aktiven Stoffen und deren Mischungen.

9. Kosmetische Verwendung einer lipophoben und homophoben HIPE-Öl-in-Wasser-Emulsion nach einem der Ansprüche 1 bis 8 als ein Träger von einer oder mehreren aktiven Substanzen, um den Durchgang der aktiven Substanzen durch eine externe physiologische Barriere eines Säugetierkörpers zu vermindern oder zu verhindern.

10. Verwendung einer lipophoben und homophoben HIPE-Öl-in-Wasser-Emulsion nach einem der Ansprüche 1 bis 9 als Kosmetikum für Haut oder Haar.

11. Verfahren zum Herstellen einer HIPE-Öl-in-Wasser-(O/W-)Emulsion mit einer öligen internen Phase, die mehr als 74 Volumen-% ausmacht, umfassend das Dispergieren eines Volumens eines Öls, das mehr als 74 % ausmacht, in einer wässrigen Lösung von Perfluorpolyetherphosphat (PFPE) der Formel
**(HO)₂OP-O-(CH₂CH₂O)ₙ-CH₂-Rf-CH₂-(OCH₂CH₂)ₙO-P-O(OH)₂**
wobei:
n 1 bis 2 beträgt,
R_{f} eine Perfluorpolyetherkette der Formel:
-(CF₂CF₂O)ₚ-(CF₂O)_{q}-
mit p/q von 0,5 bis 3,0 ist,
das durchschnittliche Molekulargewicht von R_{f} im Bereich von 500 bis 4000, bevorzugt 1000 bis 2000, stärker bevorzugt 1400 bis 1600 liegt, und wobei das Perfluorpolyetherphosphat bevorzugt Polyperfluorethoxymethoxydifluorethyl-PEG-Phosphat ist.

12. Verfahren nach Anspruch 11, **dadurch gekennzeichnet, dass** es umfasst:
einen Dispersionsschritt unter Rühren einer öligen Phase in der wässrigen Lösung von PFPE-Phosphat bis zur Bildung einer flüssigen Emulsion, und einen Einengungsschritt, der die Zugabe von Öl zu der flüssigen Emulsion unter Rühren bis zur Bildung einer ölabweisenden viskosen Emulsion umfasst.

13. Verfahren nach Anspruch 12, **dadurch gekennzeichnet, dass** der Dispersionsschritt die Zugabe von erwärmtem Öl unter Rühren zu der erwärmten Lösung von PFPE-Phosphat mit einem pH im Bereich von 3 bis 8 bis zum Erhalt einer flüssigen Emulsion umfasst, die bevorzugt 10 bis 50 Teile Öl je 100 Teile der wässrigen Phase enthält.

14. Verfahren nach Anspruch 12, **dadurch gekennzeichnet, dass** der Einengungsschritt umfasst:
Zugabe von vorgewärmtem Öl unter Rühren zu der Fluidemulsion, die in dem Dispersionsschritt erhalten wurde, bis zum Erreichen eines Anteils von 3 Teilen Öl je einem Teil wässriger Phase, und Beibehalten des Rührens bis zum Erhalt einer ölabweisenden viskosen Emulsion.

15. Verfahren nach Anspruch 11, **dadurch gekennzeichnet, dass** es umfasst:
einen Dispersionsschritt unter Rühren einer öligen Phase in der wässrigen Lösung von PFPE-Phosphat bis zur Bildung einer verdünnten suprafluiden Emulsion, und
einen Einengungsschritt der verdünnten suprafluiden Emulsion durch Abtrennung der wässrigen Phase, bevorzugt durch Zentrifugation oder durch Rotationsverdampfung bis zur Bildung einer ölabweisenden viskosen Emulsion.

16. Verfahren nach Anspruch 15, **dadurch gekennzeichnet, dass** der Dispersionsschritt umfasst:
Zugeben des vorgewärmten Öls unter Rühren zu einer vorgewärmten wässrigen Lösung von PFPE-Phosphat mit einem pH im Bereich von 3 bis 8, und
Herunterkühlen auf Raumtemperatur und Beibehalten des Rührens bis zur Bildung einer verdünnten suprafluiden Emulsion.

## Revendications

1. Émulsion d'huile-dans-l'eau (H/E) avec une phase dispersée élevée (HIPE) comprenant une phase aqueuse continue et une phase interne huileuse, dispersée dans la phase aqueuse continue, **caractérisée en ce que** la phase interne huileuse est supérieure à 74 % en volume et ladite émulsion est homophobe et lipophobe et elle comprend du phosphate de perfluoropolyéther (PFPE) de formule
**(HO)₂OP-O-(CH₂CH₂O)ₙ-CH₂-R_{f}-CH₂-(OCH₂CH₂)ₙ-O-P-O(OH)₂**
dans laquelle :
n vaut de 1 à 2
R_{f} est une chaîne de perfluoropolyéther de formule :
**-(CF₂CF₂O)ₚ-(CF₂O)_{q}-**
avec p/q de 0,5 à 3,0
le poids moléculaire moyen de R_{f} est compris dans la plage de 500 à 4 000,
de préférence dans la plage de 1 000 à 2 000, plus préférablement de 1 400 à 1 600 solubilisé dans la phase aqueuse continue.

2. Émulsion d'huile-dans-l'eau HIPE, selon la revendication 1, **caractérisée en ce que** ledit phosphate de perfluoropolyéther est du polyperfluoroéthoxyméthoxy difluoroéthyl PEG phosphate (nom INCI).

3. Émulsion d'huile-dans-l'eau HIPE, ayant une phase interne huileuse, supérieure à 74 % en volume, lipophobe et homophobe, obtenue par émulsification d'une phase huileuse ayant un volume supérieur à 74 % avec une solution aqueuse de phosphate de perfluoropolyéther (PFPE) de formule :
**(HO)₂OP-O-(CH₂CH₂O)ₙ-CH₂-R_{f}-CH₂-(OCH₂CH₂)ₙ-O-P-O(OH)₂**
dans laquelle :
n vaut de 1 à 2
R_{f} est une chaîne de perfluoropolyéther de formule :
**-(CF₂CF₂O)ₚ-(CF₂O)_{q}-**
avec p/q de 0,5 à 3,0
le poids moléculaire moyen de R_{f} est compris entre 500 et 4 000, de préférence de 1 000 à 2 000, plus préférablement de 1 400 à 1 600 ;
et par séparation de la phase aqueuse.

4. Émulsion d'huile-dans-l'eau HIPE, lipophobe et homophobe, selon la revendication 3,
**caractérisée en ce que** ledit phosphate de perfluoropolyéther est du polyperfluoroéthoxyméthoxy difluoroéthyl PEG phosphate.

5. Émulsion d'huile-dans-l'eau HIPE, lipophobe et homophobe, selon la revendication 3 ou 4, **caractérisée en ce que** ladite émulsification comprend :
la dispersion sous agitation d'une phase huileuse dans la solution aqueuse de phosphate de PFPE, jusqu'à la formation d'une émulsion fluide, et
la concentration de l'émulsion fluide par ajout d'huile sous agitation, jusqu'à la formation d'une émulsion visqueuse oléofuge.

6. Émulsion d'huile-dans-l'eau HIPE, lipophobe et homophobe, selon la revendication 3 ou 4, **caractérisée en ce que** ladite émulsification comprend :
la dispersion sous agitation d'une phase huileuse dans la solution aqueuse de phosphate de PFPE, jusqu'à la formation d'une émulsion diluée superfluide, et
la concentration de l'émulsion diluée superfluide par séparation de la phase aqueuse, jusqu'à la formation d'une émulsion visqueuse oléofuge.

7. Émulsion d'huile-dans-l'eau HIPE, lipophobe et homophobe, selon l'une quelconque des revendications 3 à 6, **caractérisée en ce que** ladite phase huileuse inclut une huile et une ou plusieurs substances actives dispersées dans celle-ci.

8. Émulsion d'huile-dans-l'eau HIPE, lipophobe et homophobe, selon la revendication 7, **caractérisée en ce que** ladite substance active est choisie parmi les filtres solaires, les parfums, les principes pharmacologiquement actifs et les mélanges de ceux-ci.

9. Utilisation cosmétique d'une émulsion d'huile-dans-l'eau HIPE, lipophobe et homophobe, selon l'une quelconque des revendications 1 à 8, en tant que vecteur d'une ou plusieurs substances actives pour réduire ou pour prévenir le passage desdites substances actives à travers une barrière physiologique externe d'un organisme de mammifère.

10. Utilisation d'une émulsion d'huile-dans-l'eau HIPE, lipophobe et homophobe, selon l'une quelconque des revendications 1 à 9 en tant que cosmétique pour la peau ou les cheveux.

11. Procédé de préparation d'une émulsion d'huile-dans-l'eau (H/E) HIPE, ayant une phase interne huileuse supérieure à 74 % en volume, comprenant la dispersion d'un volume d'une huile supérieur à 74 % dans une solution aqueuse de phosphate de perfluoropolyéther (PFPE) de formule
**(HO)₂OP-O-(CH₂CH₂O)ₙ-CH₂-Rf-CH₂-(OCH₂CH₂)ₙ-O-P-O(OH)₂**
dans laquelle :
n vaut de 1 à 2
R_{f} est une chaîne de perfluoropolyéther de formule :
**-(CF₂CF₂O)ₚ-(CF₂O)_{q}-**
avec p/q de 0,5 à 3,0
le poids moléculaire moyen de R_{f} est dans la plage de 500 à 4 000, de préférence de 1 000 à 2 000, plus préférablement de 1 400 à 1 600, et dans laquelle ledit phosphate de perfluoropolyéther est de préférence du polyperfluoroéthoxyméthoxy difluoroéthyl PEG phosphate.

12. Procédé, selon la revendication 11, **caractérisé en ce qu'**il comprend :
une étape de dispersion sous agitation d'une phase huileuse dans la solution aqueuse de phosphate de PFPE, jusqu'à la formation d'une émulsion fluide, et une étape de concentration incluant l'ajout d'huile à l'émulsion fluide sous agitation, jusqu'à la formation d'une émulsion visqueuse oléofuge.

13. Procédé selon la revendication 12, **caractérisé en ce que** l'étape de dispersion inclut l'ajout d'huile chauffée sous agitation à la solution chauffée de phosphate de PFPE, ayant un pH dans la plage de 3 à 8, jusqu'à obtention d'une émulsion fluide, contenant de préférence de 10 à 50 parties d'huile pour 100 parties de la phase aqueuse.

14. Procédé, selon la revendication 12, **caractérisé en ce que** l'étape de concentration inclut :
l'ajout d'huile préchauffée sous agitation à l'émulsion fluide obtenue dans l'étape de dispersion, jusqu'à atteindre une proportion de 3 parties d'huile pour une partie de phase aqueuse, et le maintien de l'agitation, jusqu'à obtention d'une émulsion visqueuse oléofuge.

15. Procédé, selon la revendication 11, **caractérisé en ce qu'**il inclut :
une étape de dispersion sous agitation d'une phase huileuse dans la solution aqueuse de phosphate de PFPE, jusqu'à la formation d'une émulsion diluée superfluide, et
une étape de concentration de l'émulsion diluée superfluide par séparation de la phase aqueuse, de préférence par centrifugation ou par évaporation rotative, jusqu'à la formation d'une émulsion visqueuse oléofuge.

16. Procédé, selon la revendication 15, **caractérisé en ce que** l'étape de dispersion inclut :
l'ajout, sous agitation, de l'huile préchauffée à une solution aqueuse préchauffée de phosphate de PFPE, ayant pH dans la plage de 3 à 8, et
le refroidissement à température ambiante, tout en maintenant l'agitation, jusqu'à la formation d'une émulsion diluée superfluide.
